# EUROPEAN PATENT SPECIFICATION

(11) **EP 3 934 588 B1**
(45) Date of publication and mention of the grant of the patent: **24.01.2024**
(21) Application number: 21708698.2
(22) Date of filing: 05.03.2021
(51) Int. Cl.: A61F 2/95

(54) **STENT DELIVERY SYSTEM AND HANDLING DEVICE FOR A STENT DELIVERY SYSTEM**
STENTEINFÜHRSYSTEM UND HANDHABUNGSVORRICHTUNG FÜR EIN STENTZUFÜHRUNGSSYSTEM
SYSTÈME DE MISE EN PLACE D'ENDOPROTHÈSE VASCULAIRE ET DISPOSITIF DE MANIPULATION POUR UN SYSTÈME DE MISE EN PLACE D'ENDOPROTHÈSE VASCULAIRE

(30) Priority: 26.03.2020 EP 20165757
(43) Date of publication of application: 12.01.2022
(73) Proprietor: B. Braun Melsungen AG, 34212 Melsungen (DE)
(72) Inventor: MURPHY, Brian, Bray, Co. Wicklow, A 98 Y6WO (IE)
(74) Representative: Patentanwälte Ruff, Wilhelm, Beier, Dauster & Partner mbB
(86) International application number: PCT/EP2021/055634
(87) International publication number: WO 2021/190901

(56) References cited:
- US-A1- 2010 036 472
- US-A1- 2015 057 739
- US-A1- 2015 094 794
- US-A1- 2015 250 631

## Description

### TECHNICAL FIELD AND PRIOR ART

The invention relates to a stent delivery system.

It is well known to employ intravascular endoprostheses delivered percutaneously for the treatment of diseases of various body vessels. Such intravascular endoprostheses are commonly referred to as "stents". A stent is a generally longitudinal tubular device of biocompatible material having holes or slots that define a flexible framework that allows radial expansion of the stent, by a balloon catheter or the like, or alternatively by self-expansion due to shape memory characteristics of the material within the body vessel. The flexible framework is configured to allow the stent to be compressed into a smaller outer diameter so that it can be mounted inside a stent delivery system.

The stent delivery system is used to convey the stent to a desired location within the body vessel, and then to release the stent in position. Upon release the stent may self-expand into a larger outer diameter.

WO 2019/053508 A1 discloses a stent delivery system having a handling device and a catheter arrangement. The catheter arrangement comprises an inner shaft, an outer sheath disposed coaxially to the inner shaft, and a stent which is received radially between the inner shaft and the outer sheath. The inner shaft of the catheter arrangement has a proximal end that is fixed to a housing of the handling device. The stent delivery system further comprises a flexible pull member which, with one end, engages on a proximal end of the outer sheath and, with the other end, is held on a winding spool of the handling device. The winding spool is operatively connected to a thumbwheel that is rotatably mounted inside the housing of the handling device. For release of the stent, the thumbwheel is rotated manually, whereby the winding spool is rotated. The flexible pull member engaging on the winding spool is thereby wound onto the winding spool and, as a result, the outer sheath is displaced relative to the inner shaft in the proximal direction. Due to this proximal retraction of the outer sheath, the stent is released and, thus, can expand within the blood vessel.

US 2015/0250631 A1 discloses a stent delivery system including a wire collection device which is constructed with a thumbwheel coupled to a collection spindle that is rotatable to collect a retraction wire about a varying collection diameter of the collection spindle. A proximal end of an outer stent-constraining sheath is coupled to the collection spindle by the retraction wire and a distal end of the outer sheath retractably surrounds a distally-disposed self-expanding stent. The collection spindle includes a substantially constant outer diameter greater than the varying collection diameter, and the varying collection diameter increases from a first end of the spindle towards a second end of the spindle along a spiral groove formed between the varying collection diameter and the substantially constant outer diameter in a manner that provides substantially constant rotating force on the thumbwheel, thereby accommodating changing resistance as the outer sheath is retracted and releases binding force of the stent.

US 2010/0036472 A1 discloses a delivery system utilizing a handle assembly including an actuating mechanism capable of initially providing sufficient mechanical advantage to overcome static friction when initiating deployment of the medical device. The actuating mechanism includes components which help to increase the speed of deployment as the physician continues to manipulate the actuating mechanism.

US 2015/0057739 A1 discloses a wire collection device for a stent delivery system including a plurality of nested wire collection drums with a first drum concentric in a second drum. A thumbwheel coupled to the first drum can rotate to actuate rotation of the first drum to collect the retraction wire around the diameter of the first drum. A catching mechanism can rotate about the first drum to engage a catch portion of the second drum and actuate rotation of the second drum to collect the retraction wire around the outer diameter of the second drum. This increases the collection diameter of the retraction wire to provide a mechanical advantage for sheath retraction.

US 2015/0094794 A1 discloses a stent deployment system including a wire collection device with a profiled retraction wire and an actuator coupled to a collection spindle. A distal end of the profiled retraction wire is coupled to a sledge that is coupled to an outer sheath. A proximal end of the profiled retraction wire is coupled to the collection spindle. The actuator can actuate rotation of the collection spindle to collect the profiled retraction wire around the collection spindle, and collection of the profiled retraction wire retracts the outer sheath across an outer surface of the stent so as to deploy a stent surrounded by a distal end of the outer sheath. The wire collection device has a varying collection diameter that includes the diameter of the collection spindle and the combined thickness of collected portions of the profiled retraction wire.

### SUMMARY OF THE INVENTION

It is the object of the present invention to provide a stent delivery system that allows a simplified handling during the release of the stent.

This object is solved by providing a stent delivery system having the features of claim 1.

According to the invention, a stent delivery system is provided comprising: a handling device having a housing, a thumbwheel mounted in the housing to be rotatable, and a winding spool rotating together with the thumbwheel; a catheter arrangement having an inner shaft, with a proximal end of the shaft fixed on the housing, an outer sheath disposed coaxially to the inner shaft, and having at least a first stent which is received radially between the inner shaft and the outer sheath; a flexible pull member which, with one end, engages on a proximal end of the outer sheath and, with the other end, is held on the winding spool to be windable; wherein, for release of at least the first stent, the outer sheath is displaceable relative to the inner shaft in the proximal direction by means of winding the flexible pull member on the winding spool; wherein the winding spool has different winding diameters on which the flexible pull member engages and is windable in different displacement positions of the outer sheath, whereby different transmission ratios result between the rotation movement of the thumbwheel and the proximal displacing movement of the outer sheath. Owing to the solution according to the invention, in the different displacement positions of the outer sheath, different transmission ratios are achieved between the rotation movement of the thumbwheel and the proximal displacing movement of the outer sheath resulting therefrom. Depending on the transmission ratio, the outer sheath is displaced either in comparatively rough or fine, rapid or slow, respectively, manner in the proximal direction following the rotation movement of the thumbwheel. In other words, depending on the displacement position of the outer sheath, a relatively rough or fine controlling of the displacement move is achieved by means of the thumbwheel. Hereby, simplified handling during release of at least the first stent can be achieved.

In one embodiment, the different winding diameters of the winding spool are matched to different geometric properties of the catheter arrangement. The different geometric properties of the catheter arrangement may in particular be axial distance dimensions between different components and/or sections of the catheter arrangement. As an alternative or in addition, the different geometric properties may be axial length dimensions of different components and/or sections of the catheter arrangement.

In one embodiment, the different geometric properties are a first axial distance between a distal end of the inner shaft and the first stent, a first length of the first stent, a second axial distance between the first stent and a second stent, and/or a second length of the second stent. For example, a first winding diameter of the winding spool can be matched to the first axial distance between the distal end of the inner shaft and the first stent, and a second winding diameter of the winding spool can be matched to the first length of the first stent. Upon release of the first stent, the outer sheath is initially displaced along the first axial distance and subsequently along the first length relative to the inner shaft in the proximal direction. Due to the corresponding adaptation of the winding diameters, the proximal displacement for bridging the first axial distance can be made comparatively more rapid than during bridging of the first length, or vice versa. Moreover, a second stent can be received radially between the inner shaft and the outer sheath, and spaced proximally from the first stent. As an alternative or in addition, the different winding diameters of the winding spool can be matched to the second axial distance and/or the second length of the second stent. Preferably, the different winding diameters of the winding spool are matched to the above mentioned geometric properties of the catheter arrangement such that for proximal retracting of the outer sheath along the first axial distance, the first length, the second axial distance and/or the second length are in each case exactly one revolution of the thumbwheel is required. Preferably, the first axial distance is greater than the first length and/or the second axial distance and/or the second length. In this case, the first winding diameter is configured larger than the second winding diameter such that an equal amount of rotation of the thumbwheel is required to bridge the different axial distances and/or different lengths of the catheter arrangement. Preferably, this amount of rotation is exactly 360 degrees, i.e. one revolution. In other words, in this case one and the same amount of thumb wheel rotation is required in each case to bridge the first axial distance, the first length, the second axial distance and/or the second length.

According to the invention, the different winding diameters are formed on different axial sections of the winding spool. The different axial sections are adjacent to each other in the axial direction of the winding spool. A first axial section has and/or produces a first winding diameter, and a second axial section has and/or produces a second winding diameter. By means of actuating the thumbwheel, the flexible pull member is initially wound on the first winding diameter and, subsequently, wound on the second winding diameter. According to the invention, a transition between the different winding diameters is stepped in the radial direction.

According to the invention, the winding spool has at least a first winding diameter which the flexible pull member engages during a displacement move of the outer sheath between a first displacement position and a second displacement position, and a second winding diameter on which the flexible pull member engages during a displacement move of the outer sheath between the second displacement position and a third displacement position. For release of at least the first stent, the outer sheath is moved relative to the inner shaft in the proximal direction starting from the first displacement position to the second displacement position and, subsequently, further to the third displacement position. In that context, the flexible pull member initially cooperates with the first winding diameter of the winding spool. That is, during a displacement move starting from the first displacement position to the second displacement position, the flexible pull member is wound on the first winding diameter. As a result, a first transmission ratio between the rotation movement of the thumbwheel or the winding spool rotating together with the thumbwheel, respectively, and the displacing movement of the outer sheath is obtained. Upon a further proximal displacement starting from the second displacement position to the third displacement position, the flexible pull member cooperates with the second winding diameter and is wound thereon. As a result, a corresponding second transmission ratio is obtained. Due to these different transmission ratios, the outer sheath is displaced - during a constant rotational movement of the thumbwheel and, thus, also the winding spool - comparatively more rapidly between the first displacement position and the second displacement position than between the second displacement position and the third displacement position. Preferably, a first axial distance between the first and the second position and a second axial distance between the second and third position are matched to the first winding diameter and the second winding diameter such that the same amount of thumb wheel rotation, preferably exactly one revolution, is required to retract the outer sheath along the first and the second axial distance.

According to the invention, the winding spool has a helical circumferential groove, with the groove bottom thereof providing the first winding diameter. During winding on the first winding diameter the flexible pull member is guided within the helical circumferential groove. Preferably, the helical circumferential groove extends over 360° along the circumference of the winding spool. According to the invention, the first winding diameter is larger than the second winding diameter. The helical circumferential groove is disposed on a first axial section of the winding spool. Preferably, the helical circumferential groove is configured such that exactly one revolution of the thumbwheel is required to initially retract the outer sheath - starting from a delivery condition of the stent delivery system and/or the catheter arrangement - to the distal end of the first stent.

According to the invention, the winding spool has a cylindrical section, with the outer diameter thereof providing the second winding diameter. The cylindrical section preferably has a circular cylindrical design. According to the invention, the second winding diameter is smaller than the first winding diameter. The cylindrical section is disposed on a second axial section of the winding spool. Preferably, the cylindrical section is configured such that exactly one revolution of the thumbwheel is required to further retract the outer sheath - starting from the distal end of the first stent - to release the first stent and, in case further stents are provided, each further stent.

### BRIEF DESCRIPTION OF THE DRAWINGS

In the following, an embodiment of the invention will be described in detail with reference to the drawings. Throughout the drawings, the same elements will be denoted by the same reference numerals. The drawings schematically show:
- Fig. 1: in an isometric view an embodiment of a stent delivery system according to the invention, including a handling device and a catheter arrangement with a plurality of stents, wherein the catheter arrangement is illustrated with partial sections;
- Fig. 2: the stent delivery system according to Fig. 1 in the region of the handling device in a further isometric view;
- Fig. 3: the stent delivery system according to Figs. 1 and 2 in a view corresponding to Fig. 2 with individual components and/or sections of the handling device hidden in the drawing;
- Fig. 4: an enlarged detail illustration of the view according to Fig. 3 in the region of a thumbwheel and a winding spool operatively connected to the thumbwheel;
- Fig. 5, 6: the winding spool in a radial direction of viewing (Fig. 6) and in a longitudinal section V-V (Fig. 5) according to Fig. 6; and
- Fig. 7: another isometric view similar to Fig. 3 and additionally showing a flexible pull member which engages, with one end, on the catheter arrangement and, with the other end, is held on the winding spool to be windable.

### DETAILED DESCRIPTION OF EMBODIMENTS OF THE INVENTION

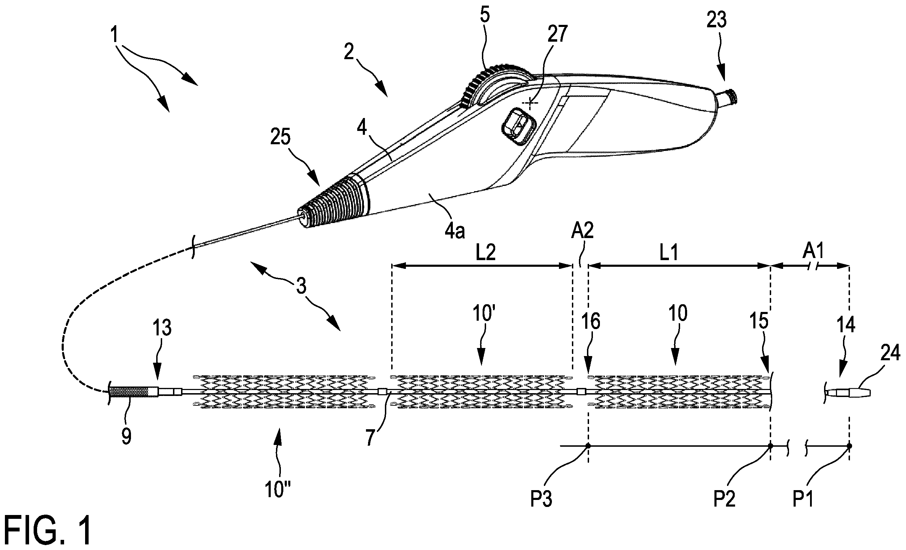

According to Fig. 1, a stent delivery system 1 comprises a handling device 2 and a catheter arrangement 3. The stent delivery system 1 is intended for use in treatment of stenosis in blood vessels.

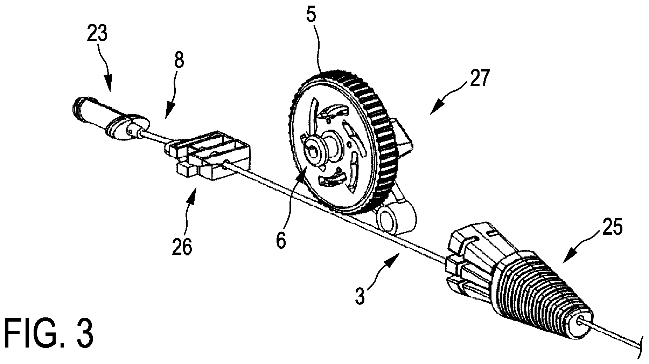

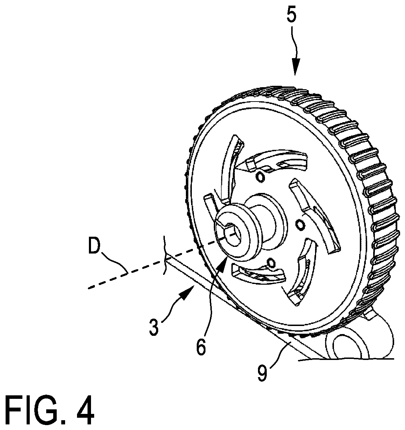

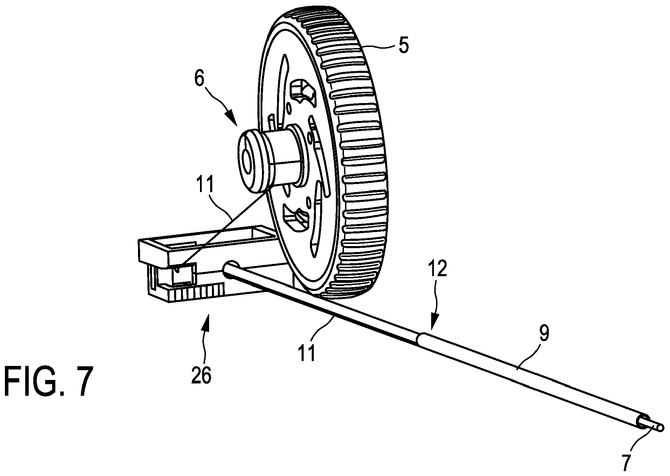

The handling device 2 has a housing 4 with a thumbwheel 5 mounted in the housing 4 to be rotatable and a winding spool 6 rotating together with the thumbwheel 5 (Fig. 3, 4). The catheter arrangement 3 has an inner shaft 7 and an outer sheath 9 disposed coaxially to the inner shaft 7. A proximal end 8 of the inner shaft 7 is at least indirectly fixed on the housing 4 in a generally well-known manner. Further, the catheter arrangement 3 has at least a first stent 10 which is received radially between the inner shaft 7 and the outer sheath 9 in a condition of the catheter arrangement not illustrated in more detail in the drawing. Moreover, the stent delivery system 1 includes a flexible pull member 11 (Fig. 7) which, with one end, engages on a proximal end 12 of the outer sheath 9 and, with the other end, is held on the winding spool 6 to be windable. For release of at least the first stent 10, the outer sheath 9 is displaceable relative to the inner shaft 7 in the proximal direction by means of winding the flexible pull member 11 on the winding spool 6. The above mentioned release takes place starting from a displacement position of the outer sheath 9 not illustrated in more detail in the drawing, wherein a distal end 13 of the outer sheath 9 is essentially flush with a distal end 14 of the inner shaft. In this condition, the first stent 10 is compressed in the radial direction between an outer shell surface of the inner shaft 7 and an inner shell surface of the outer sheath 9. Following the above mentioned retraction of the outer sheath 9 in the proximal direction, the first stent 10 is released in the radial direction, and thereby may expand in the radial direction manner. With reference to Fig. 1, a corresponding released and expanded condition of the first stent 10 is illustrated.

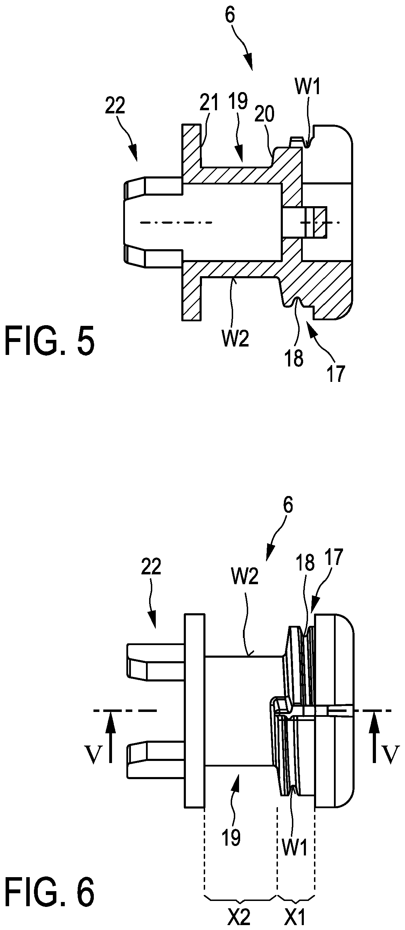

As shown in particular with reference to Figs. 5 and 6, the winding spool 6 has different winding diameters W1, W2 on which the flexible pull member 11 engages and is windable in different displacement positions of the outer sheath 9, whereby different transmission ratios result between the rotation movement of the thumbwheel 5 and the proximal displacing movement of the outer sheath 9.

In the embodiment shown, the winding spool 6 has two different winding diameters W1, W2, namely a first winding diameter W1 and a second winding diameter W2.

For release of the first stent 10, the outer sheath 9 is moved relative to the inner shaft 7 starting from a first displacement position P1 in the proximal direction to a second displacement position P2 and, subsequently, further in the proximal direction to at least one third displacement position P3. The displacement positions P1, P2, P3 are schematically demonstrated with reference to Fig. 1. In the embodiment shown, the flexible pull member 11 cooperates with the first winding diameter W1 during a displacement of the outer sheath 9 between the first displacement position P1 and the second displacement position P2. That is, the flexible pull member 11 initially engages on the first winding diameter W1 and is wound thereon. During a further displacement of the outer sheath 9 from the second displacement position P2 to the third displacement position P3, the flexible pull member 11 cooperates with the second winding diameter W2. That is, the flexible pull member 11 engages on the second winding diameter W2 and is wound thereon. As a result, said different transmission ratios are obtained, namely a first transmission ratio during the displacement between the first displacement position P1 and the second displacement position P2, and a second transmission ratio during the further displacement between the second displacement position P2 and the third displacement position P3.

In the embodiment shown, the outer sheath 9, the inner shaft 7 and the first stent 10 are positioned in relation to each other in the different displacement positions of the outer sheath 9 as follows: In the first displacement position P1, the distal end 13 of the outer sheath 9 is oriented flush to the distal end 14 of the inner shaft 7. In the second displacement position P2, the distal end 13 of the outer sheath 9 is retracted relative to the distal end 14 of the inner shaft 7 in the proximal direction and is essentially flush with a distal end 15 of the first stent 10. In the third displacement position P3, the distal end 13, starting from the second displacement position P2, is retracted further in the proximal direction and is essentially flush with a proximal end 16 of the first stent 10. Correspondingly, the first stent 10 is received radially between the inner shaft 7 and the outer sheath 9, both in the first displacement position P1 and in the second displacement position P2. In contrast, in the third displacement position P3, the first stent 10 is released.

In the embodiment shown, the different winding diameters W1, W2 of the winding spool 6 are matched to different geometric properties of the catheter arrangement 3.

These geometric properties of the catheter arrangement 3 are, in the embodiment shown, a first axial distance A1 and a first length L1. The first axial distance A1 extends in the axial direction between the distal end 14 of the inner shaft 7 and the first stent 10, more precisely the distal end 15 thereof. In the present case, the first length L1 is the length of the first stent 10. Herein, the first winding diameter W1 is matched to the first axial distance A1 and the second winding diameter W2 is matched to the first length L1. In the present case, this matching is such that for displacing the outer sheath 9 along the first axial distance A1 and for bridging the first length L1, in each case exactly one revolution of the thumbwheel 5 and, thus, also of the winding spool 6 are required. For that purpose the first winding diameter W1 has a perimeter which corresponds to the axial distance A 1 and the second winding diameter W2 has a perimeter which corresponds to the first length L1 of the first stent 10.

In the embodiment shown, the catheter arrangement 3 has further stents 10', 10", in addition to the first stent 10, namely a second stent 10' and a third stent 10". Of course, such a configuration is not mandatory. The second stent 10' and the third stent 10" are received in the radial direction between the inner shaft 7 and the outer sheath 9, in a manner corresponding to the first stent 10, again not illustrated herein, and releasable by means of a displacement of the outer sheath 9 passing beyond the third displacement position P3 in the proximal direction.

In a not shown embodiment, as an alternative or in addition, the different winding diameters of the winding spool are matched to a second axial distance A2 between the first stent 10 and the second stent 10' and/or a second length L2 of the second stent 10'.

Before discussing the further specific configuration of the handling device 2 and the catheter arrangement 3, the further features of the winding spool 6 are explained, in particular with reference to Figs. 5 and 6:
The figures show that the different winding diameters W1, W2 are provided on different axial sections X1, X2 of the winding spool 6. The first winding diameter W1 is formed on a first axial section X1 and the second winding diameter W2 is formed on a second axial section X2 of the winding spool 6. The axial sections X1, X2 are disposed adjacent to each other in the axial direction of the winding spool 6. In the present case, during the proximal displacement of the outer sheath 9 starting from the first displacement position P1, the flexible pull member 11 cooperates initially with the first axial section X1. Subsequently, namely after reaching the second displacement position P2 of the outer sheath 9, the flexible pull member 11 cooperates with the second axial section X2. Put in other words, the flexible pull member 11 is initially wound on the first winding diameter W1 of the first axial section X1 and then enters, namely after reaching the second displacement position P2 of the outer sheath 9, the second axial section X2 and there is wound on the second winding diameter W2.

Further, the winding spool 6 has a helical circumferential groove 17, with the groove bottom 18 thereof providing the first winding diameter W1. The circumferential groove 17 extends along the circumference of the first axial section X1 and has a pitch oriented in the axial direction of the winding spool 6, as a result of which said helical shape of the circumferential groove 17 is obtained. This configuration allows in particular precise guiding of the flexible pull member 11 during winding on the first winding diameter W1.

Further, the winding spool 6 has a cylindrical section 19, with the outer diameter thereof providing the second winding diameter W2. The cylindrical section 19 has a straight circular cylinder shape, in the present case.

In the embodiment shown, the first winding diameter W1 is larger than the second winding diameter W2. Apart from that, an outer diameter of the thumbwheel 5, not described in more detail, is greater than the first winding diameter W1.

A first radial shoulder 20 is disposed in the axial direction between the first axial section X1 and the second axial section X2. The first radial shoulder 20 forms a step between the first winding diameter W1 and the second winding diameter W2 such that, during winding of the flexible pull member 11, a quasi-abrupt transition results, and not a continuous variation of the transmission ratio.

Moreover, in the present case, the winding spool 6 has a second radial shoulder 21 which forms an axial boundary of the second axial section X2 and, thus, also of the cylindrical section 19. In this respect, the second axial section X2 extends in the axial direction between the two shoulders 20, 21.

In the present case, the winding spool 6 moreover has a fastening section 22 which is provided for torque-transmitting fastening of the winding spool 6 on the thumbwheel 5, and cooperates with a complementary fastening section of the thumbwheel 5, not illustrated in more detail. The fastening section 22 is disposed on a front end region of the winding spool 6 facing away from the first winding diameter W1.

Further details of the configuration of the handling device 2 and the catheter arrangement 3 will be discussed below, however, are not to be considered as mandatory features in view of the present invention.

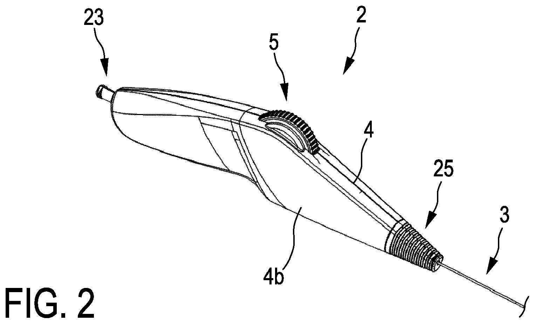

In the present case, the housing 4 has two housing halves 4a, 4b, namely a first housing half 4a (Fig. 1) and a second housing half 4b. In the ready-for-use condition of the stent delivery system 1, shown with reference to the Figs. 1 and 2, the housing halves 4a, 4b are joined together in a generally well-known manner. In the present case, the housing 4 is manufactured from a dimensionally stable synthetic material. As is further apparent with reference to the Figs. 1 and 2, the housing 4 provides a handle which may be grasped by a hand such that the thumb of the respective hand is placed in the region of an upper side of the housing 4 for rotating actuation of the thumbwheel 5.

The thumbwheel 5 is mounted in the housing 4 to be rotatable about a rotation axis D (Fig. 4) and protrudes, in the radial direction of the thumbwheel 5, from a recess of the housing 4 not described in more detail. As a result, the thumbwheel 5 is manually operable in the above described manner. The rotation axis D is perpendicular to the proximal displacement direction of the outer sheath 9 and - during conventional handling - in approximately horizontal orientation, in the embodiment shown.

The winding spool 6 is oriented coaxially to the thumbwheel 5 and connected to the thumbwheel 5 by the fastening section 22 (Fig. 5) in a torque-transmitting manner. As a result, the winding spool 6 is rotatable together with the thumbwheel 5 about the rotation axis D. In a not shown embodiment, the winding spool 6 can be provided integrally with the thumbwheel 5. In the embodiment shown, the winding spool 6 is disposed completely inside the housing 4.

The catheter arrangement 3 extends in the region of the handling device 2 between a distal end and a proximal end of the housing 4 within the latter. The inner shaft 7 includes a lumen, not apparent in more detail, which extends between the proximal end 8 and the distal end 14. In the region of the proximal end 8, the lumen leads into a Luer port 23 which is fixed to the proximal end of the housing 4 in a generally well-known manner. On its distal end 14, the inner shaft 7 includes a catheter tip 24. The inner shaft 7 has a flexible design. The outer sheath 9 is manufactured from a braided material and connected in the region of its proximal end 12 (Fig. 7) to the flexible pull member 11 for transfer of pulling force. In the embodiment shown, the first stent 10 is a self-expanding stent manufactured and designed in a manner known to the skilled person. The same applies to the second stent 10' and the third stent 10".

Further, in the present case, the handling device 2 includes a guiding tip 25 which is disposed on a distal end of the housing 4 and is held between the housing halves 4a, 4b. The catheter arrangement 3 extends, in the region of the distal end of the housing 4, through the guiding tip 25 in the axial direction.

Furthermore, the handling device 2 includes a deflecting and tensioning device 26 which is disposed in the axial direction of the catheter arrangement 3 between the thumbwheel 5 - and, thus, also the winding spool 6 - and the proximal end 8 of the inner shaft 7. The deflecting and tensioning device 26 is intended for deflecting and tensioning of the flexible pull member 11 (Fig. 7). Moreover, a locking and unlocking mechanism 27 is provided which is intended for locking and unlocking the rotational mobility of the thumbwheel 5 and, thus, also the winding spool 6. Neither the deflecting and tensioning device 26 nor the locking and unlocking mechanism 27 are mandatory, therefore, a more detailed description thereof is omitted for reasons of brevity.

## Claims

1. Stent delivery system (1), comprising:
- a handling device (2) having a housing (4), a thumbwheel (5) mounted in the housing (4) to be rotatable, and a winding spool (6) rotating together with the thumbwheel (5);
- a catheter arrangement (3) having an inner shaft (7), with a proximal end (8) of the inner shaft (7) fixed on the housing (4), an outer sheath (9) disposed coaxially to the inner shaft (7), and having at least a first stent (10) which is received radially between the inner shaft (7) and the outer sheath (9);
- a flexible pull member (11) which, with one end, engages on a proximal end (12) of the outer sheath (9) and, with the other end, is held on the winding spool (6) to be windable;
- wherein, for release of at least the first stent (10), the outer sheath (9) is displaceable relative to the inner shaft (7) in the proximal direction by means of winding the flexible pull member (11) on the winding spool (6);
- wherein the winding spool (6) has different winding diameters (W1, W2) on which the flexible pull member (11) engages and is windable in different displacement positions (P1, P2, P3) of the outer sheath (9), whereby different transmission ratios result between the rotation movement of the thumbwheel (5) and the proximal displacing movement of the outer sheath (9);
- wherein the winding spool (6) has at least a first winding diameter (W1) formed on a first axial section (X1) on which the flexible pull member (11) engages during an initial displacement move of the outer sheath (9) between a first displacement position (P1) and a second displacement position (P2), and a second winding diameter (W2) formed on a second axial section (X2) on which the flexible pull member (11) engages during a subsequent displacement move of the outer sheath (9) between the second displacement position (P2) and a third displacement position (P3);
- wherein the winding spool (6) has a helical circumferential groove (17), with the groove bottom (18) thereof providing the first winding diameter (W1), wherein the winding spool (6) has a cylindrical section (19), with the outer diameter thereof providing the second winding diameter (W2), and wherein the first winding diameter (W1) is larger than the second winding diameter (W2);
- **characterized in that** a first radial shoulder (20) is disposed in axial direction between the first axial section (X1) and the second axial section (X2), the first radial shoulder (20) forming a step between the first winding diameter (W1) and the second winding diameter (W2), thereby causing, during winding of the flexible pull member (11), a quasi-abrupt and non-continuous transition of the transmission ratio.

2. Stent delivery system (1) according to claim 1, **characterized in that** the different winding diameters (W1, W2) of the winding spool (6) are matched to different geometric properties (A1, A2, L1, L2) of the catheter arrangement (3).

3. Stent delivery system (1) according to claim 2, **characterized in that** the different geometric properties are a first axial distance (A1) between a distal end (14) of the inner shaft (7) and the first stent (10), a first length (L1) of the first stent (10), a second axial distance (A2) between the first stent (10) and a second stent (10'), and/or a second length (L2) of the second stent (10').

4. Stent delivery system (1) according to any of the preceding claims, **characterized in that** the helical circumferential groove (17) is configured such that exactly one revolution of the thumbwheel (5) is required to retract the outer sheath (9) from the first displacement position (P1) to the second displacement position (P2) in order to retract the outer sheath (9) to a distal end (15) of the first stent (10).

5. Stent delivery system (1) according to any of the preceding claims, **characterized in that** the cylindrical section (19) is configured such that exactly one revolution of the thumbwheel (5) is required to retract the outer sheath (9) from the second displacement position (P2) to the third displacement position (P3) in order to release the first stent (10).

## Patentansprüche

1. Stentzuführsystem (1), umfassend:
- eine Handhabungsvorrichtung (2) mit einem Gehäuse (4), einem drehbar in dem Gehäuse (4) montierten Daumenrad (5) und einer sich gemeinsam mit dem Daumenrad (5) drehenden Wickelspule (6),
- eine Katheteranordnung (3) mit einem inneren Schaft (7), wobei ein proximales Ende (8) des inneren Schafts (7) an dem Gehäuse (4) befestigt ist, eine koaxial zu dem inneren Schaft (7) angeordnete äußere Hülse (9) und mit mindestens einem ersten Stent (10), der radial zwischen dem inneren Schaft (7) und der äußeren Hülse (9) aufgenommen ist,
- ein flexibles Zugglied (11), das mit einem Ende an einem proximalen Ende (12) der äußeren Hülse (9) in Eingriff kommt und mit dem anderen Ende an der Wickelspule (6) gehalten wird, so dass es wickelbar ist,
- wobei die äußere Hülse (9) zur Freigabe mindestens des ersten Stents (10) durch Wickeln des flexiblen Zugglieds (11) auf die Wickelspule (6) bezüglich des inneren Schafts (7) in der proximalen Richtung verschiebbar ist,
- wobei die Wickelspule (6) verschiedene Wickeldurchmesser (W1, W2) hat, an denen das flexible Zugglied (11) in Eingriff kommt und in verschiedenen Verschiebepositionen (P1, P2, P3) der äußeren Hülse (9) wickelbar ist, wodurch sich unterschiedliche Übersetzungsverhältnisse zwischen der Drehbewegung des Daumenrads (5) und der proximalen Verschiebebewegung der äußeren Hülse (9) ergeben,
- wobei die Wickelspule (6) mindestens einen ersten Wickeldurchmesser (W1), der an einem ersten axialen Abschnitt (X1) gebildet ist, an dem das flexible Zugglied (11) während einer anfänglichen Verschiebungsbewegung der äußeren Hülse (9) zwischen einer ersten Verschiebeposition (P1) und einer zweiten Verschiebeposition (P2) in Eingriff kommt, und einen zweiten Wickeldurchmesser (W2) hat, der an einem zweiten axialen Abschnitt (X2) gebildet ist, an dem das flexible Zugglied (11) während einer nachfolgenden Verschiebungsbewegung der äußeren Hülse (9) zwischen der zweiten Verschiebeposition (P2) und einer dritten Verschiebeposition (P3) in Eingriff kommt,
- wobei die Wickelspule (6) eine spiralförmige Umfangsnut (17) hat, deren Nutboden (18) den ersten Wickeldurchmesser (W1) bereitstellt, wobei die Wickelspule (6) einen zylindrischen Abschnitt (19) hat, dessen Außendurchmesser den zweiten Wickeldurchmesser (W2) bereitstellt, und wobei der erste Wickeldurchmesser (W1) größer als der zweite Wickeldurchmesser (W2) ist,
- **dadurch gekennzeichnet, dass** eine erste radiale Schulter (20) in axialer Richtung zwischen dem ersten axialen Abschnitt (X1) und dem zweiten axialen Abschnitt (X2) angeordnet ist, wobei die erste radiale Schulter (20) eine Stufe zwischen dem ersten Wickeldurchmesser (W1) und dem zweiten Wickeldurchmesser (W2) bildet, wodurch während des Wickelns des flexiblen Zugglieds (11) ein quasi abrupter und nicht durchgängiger Übergang des Übersetzungsverhältnisses veranlasst wird.

2. Stentzuführsystem (1) nach Anspruch 1, **dadurch gekennzeichnet, dass** die verschiedenen Wickeldurchmesser (W1, W2) der Wickelspule (6) zu verschiedenen geometrischen Eigenschaften (A1, A2, L1, L2) der Katheteranordnung (3) passen.

3. Stentzuführsystem (1) nach Anspruch 2, **dadurch gekennzeichnet, dass** die verschiedenen geometrischen Eigenschaften ein erster axialer Abstand (A1) zwischen einem distalen Ende (14) des inneren Schafts (7) und dem ersten Stent (10), eine erste Länge (L1) des ersten Stents (10), ein zweiter axialer Abstand (A2) zwischen dem ersten Stent (10) und einem zweiten Stent (10') und/oder eine zweite Länge (L2) des zweiten Stents (10') sind.

4. Stentzuführsystem (1) nach einem der vorhergehenden Ansprüche, **dadurch gekennzeichnet, dass** die spiralförmige Umfangsnut (17) so ausgestaltet ist, dass zum Zurückziehen der äußeren Hülse (9) aus der ersten Verschiebeposition (P1) in die zweite Verschiebeposition (P2) zum Zurückziehen der äußeren Hülse (9) zu einem distalen Ende (15) des ersten Stents (10) genau eine Umdrehung des Daumenrads (5) notwendig ist.

5. Stentzuführsystem (1) nach einem der vorhergehenden Ansprüche, **dadurch gekennzeichnet, dass** der zylindrische Abschnitt (19) so ausgestaltet ist, dass zum Zurückziehen der äußeren Hülse (9) aus der zweiten Verschiebeposition (P2) in die dritte Verschiebeposition (P3) zum Freigeben des ersten Stents (10) genau eine Umdrehung des Daumenrads (5) notwendig ist.

## Revendications

1. Système de pose d'endoprothèse (1), comprenant :
- un dispositif de manipulation (2) comportant un boîtier (4), une molette (5) montée dans le boîtier (4) pour être rotative, et une bobine d'enroulement (6) tournant avec la molette (5) ;
- un dispositif de cathéter (3) comportant un arbre interne (7), avec une extrémité proximale (8) de l'arbre interne (7) fixée sur le boîtier (4), une gaine externe (9) disposée coaxialement à l'arbre interne (7), et comportant au moins une première endoprothèse (10) qui est reçue radialement entre l'arbre interne (7) et la gaine externe (9) ;
- un élément de traction flexible (11) qui, à une extrémité, vient en prise sur une extrémité proximale (12) de la gaine externe (9) et, à l'autre extrémité, est maintenu sur la bobine d'enroulement (6) pour pouvoir être enroulé ;
- pour libérer au moins la première endoprothèse (10), la gaine externe (9) pouvant être déplacée par rapport à l'arbre interne (7) dans la direction proximale au moyen de l'enroulement de l'élément de traction flexible (11) sur la bobine d ' enroulement (6) ;
- la bobine d ' enroulement (6) ayant différents diamètres d'enroulement (W1, W2) sur lesquels l'élément de traction flexible (11) vient en prise et peut être enroulé dans différentes positions de déplacement (P1, P2, P3) de la gaine externe (9), ce qui permet d'obtenir différents rapports de transmission entre le mouvement de rotation de la molette (5) et le mouvement de déplacement proximal de la gaine externe (9) ;
- la bobine d'enroulement (6) ayant au moins un premier diamètre d ' enroulement (W1) formé sur une première section axiale (X1) sur laquelle l'élément de traction flexible (11) vient en prise pendant un mouvement de déplacement initial de la gaine externe (9) entre une première position de déplacement (P1) et une deuxième position de déplacement (P2), et un second diamètre d'enroulement (W2) formé sur une seconde section axiale (X2) sur laquelle 1 ' élément de traction flexible (11) vient en prise pendant un mouvement de déplacement ultérieur de la gaine externe (9) entre la deuxième position de déplacement (P2) et une troisième position de déplacement (P3) ;
- la bobine d'enroulement (6) ayant une rainure circonférentielle hélicoïdale (17), dont le fond de la rainure (18) fournit le premier diamètre d ' enroulement (W1), la bobine d'enroulement (6) ayant une section cylindrique (19), dont le diamètre extérieur fournit le second diamètre d'enroulement (W2), et le premier diamètre d'enroulement (W1) étant plus grand que le second diamètre d'enroulement (W2) ;
- **caractérisé en ce qu** ' un premier épaulement radial (20) est disposé dans la direction axiale entre la première section axiale (X1) et la seconde section axiale (X2), le premier épaulement radial (20) formant une marche entre le premier diamètre d'enroulement (W1) et le second diamètre d'enroulement (W2), provoquant ainsi, pendant l'enroulement de l'élément de traction flexible (11), une transition quasi-abrupte et non-continue du rapport de transmission.

2. Système de pose d ' endoprothèse (1) selon la revendication 1, **caractérisé en ce que** les différents diamètres d ' enroulement (W1, W2) de la bobine d ' enroulement (6) sont adaptés aux différentes propriétés géométriques (A1, A2, L1, L2) de l ' agencement de cathéter (3).

3. Système de pose d'endoprothèse (1) selon la revendication 2, **caractérisé en ce que** les différentes propriétés géométriques sont une première distance axiale (A1) entre une extrémité distale (14) de l'arbre interne (7) et la première endoprothèse (10), une première longueur (L1) de la première endoprothèse (10), une seconde distance axiale (A2) entre la premi è re endoprothèse (10) et une seconde endoprothèse (10'), et/ou une seconde longueur (L2) de la seconde endoprothèse (10').

4. Système de pose d'endoprothèse (1) selon l'une quelconque des revendications précédentes, **caractérisé en ce que** la rainure circonférentielle hélicoïdale (17) est configurée de telle sorte qu'exactement un tour de la molette (5) est nécessaire pour rétracter la gaine externe (9) de la première position de déplacement (P1) à la deuxième position de déplacement (P2) afin de rétracter la gaine externe (9) jusqu' à une extrémité distale (15) de la première endoprothèse (10).

5. Système de pose d'endoprothèse (1) selon l'une quelconque des revendications précédentes, **caractérisé en ce que** la section cylindrique (19) est configurée de telle sorte qu'exactement un tour de la molette (5) est nécessaire pour rétracter la gaine externe (9) de la deuxième position de déplacement (P2) à la troisième position de déplacement (P3) afin de libérer la première endoprothèse (10).
